Europäisches Patentamt

European Patent Office

Office européen des brevets

(19)

(11) EP 0 578 539 B1

(12) **FASCICULE DE BREVET EUROPEEN**

(45) Date de publication et mention
de la délivrance du brevet:
**02.09.1998 Bulletin 1998/36**

(51) Int Cl.6: **G01N 29/02**, G01N 33/04

(21) Numéro de dépôt: **93401695.7**

(22) Date de dépôt: **30.06.1993**

(54) **Dispositif et procédé de caractérisation ou de mesure par ultrasons de texture de produits**

Vorrichtung und Verfahren zur Charakterisierung oder Messung der Struktur von Produkten mit Ultraschall

Device and process for ultrasonic characterisation of measurement of the texture of products

(84) Etats contractants désignés:
**AT BE CH DE ES GB IT LI LU NL PT**

(30) Priorité: **03.07.1992 FR 9208220**

(43) Date de publication de la demande:
**12.01.1994 Bulletin 1994/02**

(73) Titulaire: **BONGRAIN S.A.**
**F-78283 Guyancourt Cédex (FR)**

(72) Inventeurs:
• **Beudon, Didier**
**F-78120 Rambouillet (FR)**
• **Audidier, Yves**
**F-91370 Verrière le Buisson (FR)**
• **Frouin, André**
**F-78000 Versailles (FR)**
• **Innocent, Jean-Pierre**
**F-78000 Versailles (FR)**

(74) Mandataire: **Orès, Bernard et al**
**Cabinet ORES**
**6, Avenue de Messine**
**75008 Paris (FR)**

(56) Documents cités:
WO-A-84/04167       WO-A-89/10559
DE-A- 1 473 707       GB-A- 2 027 201
GB-A- 2 160 974

## Description

L'invention se rapporte principalement à un dispositif et à un procédé de caractérisation ou de mesure, par ultrasons de texture de produits, notamment des produits alimentaires tels que des fromages.

On connaît des dispositifs pour déterminer les propriétés physiques d'un milieu par mesure de la vitesse de propagation d'ondes ultrasonores. Ces dispositifs n'ont jamais abouti à une mesure efficace de la texture d'un produit susceptible d'être mis en oeuvre dans l'industrie agro-alimentaire.

WO-A-8 404 167 décrit la détection de la variation de l'impédance acoustique d'un milieu. Cette mesure est effectuée par un barreau, par exemple en verre, dont une extrémité est munie d'un transducteur électroacoustique générant des ondes ultrasonores. Seule la propagation des ondes ultrasonores dans le barreau est étudiée, les ondes ultrasonores pénétrant dans le milieu n'étant pas détectées.

GB-A-2 160 974 met en oeuvre un guide d'ondes ultrasonores traversant un milieu à étudier. L'atténuation des ondes ultrasonores entre un transducteur d'émission placé à une extrémité et un transducteur de réception placé à l'extrémité opposée, permet de déterminer les propriétés du milieu. La variation de l'atténuation des ondes reçues dans le guide d'ondes est une fonction de la variation de l'impédance acoustique du milieu et de sa différence par rapport à l'impédance acoustique du guide d'ondes, le rayonnement de ce dernier dans le milieu à étudier constituant les pertes provoquant cette atténuation.

L'on connaît également des appareils d'imagerie médicale à ultrasons permettant de visualiser la structure d'un tissu. Notamment, le Brevet français 87 07795 décrit un dispositif d'échographie comportant un écran interposé entre la sonde d'émission-réception d'un faisceau d'ultrasons et un corps dont on veut obtenir une image, cet écran générant les retards de phases aléatoires en divers points du faisceau. Le déplacement de l'écran par rapport au faisceau permet d'éliminer le bruit provenant des éléments diffusant l'énergie ultrasonore autres que les éléments de structure à visualiser. Ce document indique la possibilité de caractérisation de tissus ou le contrôle non destructif des produits agro-alimentaires tels que des produits laitiers, notamment des fromages, et des viandes. Toutefois, le seul exemple de réalisation décrit un appareil d'imagerie qui élimine l'information caractéristique des tissus, en tant que bruit indésirable.

On sait qu'un des objectifs des industries agro-alimentaires est la maîtrise de leur production pour améliorer et standardiser la qualité de leurs produits.

Un des paramètres clé d'une production agro-alimentaire est la maîtrise de la texture, et cela le plus tôt possible dans la vie du produit. On entend par texture, les aspects rhéologiques et les structures micro ou macroscopiques des produits.

La technique la plus souvent utilisée est l'estimation manuelle de la texture par des techniciens spécialement entraînés.

Cette méthode repose sur la dextérité et la sensibilité des techniciens. Elle n'est pas reproductible d'une personne à l'autre et ne touche qu'un nombre réduit de produits. Elle présente toutefois l'avantage d'être non destructive.

L'on connaît d'autre part des systèmes de mesure de dureté des fromages par la technique de la pénétration uni-axiale à vitesse constante, non constante et cycles. Cette technique altère le produit et pose, de plus, les problèmes d'hygiène.

C'est par conséquent un but de la présente invention d'offrir un dispositif de mesure ou de caractérisation rapide de texture d'un produit.

C'est également un but de la présente invention d'offrir un procédé de mesure ou de caractérisation rapide de la texture d'un produit.

C'est aussi un but de la présente invention d'offrir un dispositif de mesure ou de caractérisation de texture non destructif.

C'est également un but de la présente invention d'offrir un procédé de mesure ou de caractérisation de texture non destructif.

C'est aussi un but de la présente invention d'offrir un dispositif de mesure ou de caractérisation de texture hygiénique.

C'est également un but de la présente invention d'offrir un procédé de mesure ou de caractérisation de texture d'un produit ne risquant pas de compromettre l'hygiène du produit mesuré ou caractérisé.

C'est aussi un but de la présente invention d'offrir un dispositif de mesure automatique de texture susceptible d'être mis en oeuvre sur une chaîne de production.

C'est également un but de la présente invention d'offrir un procédé de fabrication d'un produit visqueux, élastique, viscoélastique ou solide ou passant à un stade de la fabrication par un état visqueux, élastique, viscoélastique ou solide, assurant l'asservissement d'au moins un paramètre du procédé de fabrication à une mesure ou à une caractérisation de la texture du produit.

C'est aussi un but de la présente invention d'offrir un dispositif de mesure ou de caractérisation de texture ayant un bon rapport signal/bruit.

C'est également un but de la présente invention d'offrir un dispositif de mesure ou de caractérisation de texture donnant des résultats fidèles, indépendamment des variations de la température.

C'est aussi un but de la présente invention d'offrir un procédé de mesure ou de caractérisation de la quantité et de la taille des ouvertures, des hétérogénéités ou de la présence de particules solides.

C'est également un but de la présente invention d'offrir un procédé de contrôle de la qualité d'un produit par mesure ou par caractérisation de sa texture assurant le marquage des produits contrôlés.

C'est aussi un but de la présente invention d'offrir un procédé de caractérisation de texture affichant directement, quasi instantanément, un résultat de la caractérisation exploitable par un technicien.

Ces buts seront avantageusement atteints par la mesure de l'atténuation en fonction de la profondeur de pénétration des ultrasons, dans un produit dont on veut mesurer ou caractériser la texture.

L'invention a principalement pour objet un dispositif de mesure et/ou de caractérisation de la texture d'un corps, caractérisé en ce qu'il comporte une sonde d'émission et de réception d'ondes ultrasonores, reliée à un émetteur pour générer un signal électrique converti en ondes ultrasonores par la sonde et un récepteur des ondes ultrasonores émanant dudit corps et captées par la sonde ainsi que des moyens de traitement du signal pour déterminer l'atténuation dans ledit corps des ondes ultrasonores en fonction du temps de réception et donc de la profondeur de pénétration dans le corps et pour en déduire la texture, notamment la dureté dudit corps.

L'invention a également pour objet un dispositif, caractérisé en ce que la sonde comporte, en outre, des moyens de mesure de la température du corps et en ce que les moyens de traitement du signal tiennent compte de la température du corps lors de la détermination de la texture à partir des résultats de la mesure d'absorption des ondes ultrasonores.

L'invention a également pour objet un dispositif, caractérisé en ce que les moyens de traitement du signal calculent, à partir d'une succession de signaux ultrasonores captés par la sonde, une succession d'enveloppes des signaux reçus dudit corps pour en déduire une enveloppe moyenne à partir de laquelle on détermine l'atténuation des ondes ultrasonores dans ledit corps.

L'invention a également pour objet un dispositif, caractérisé en ce que le dispositif de traitement du signal sélectionne automatiquement une fenêtre temporelle dans le signal capté par la sonde ayant le meilleur rapport signal/bruit.

L'invention a également pour objet un dispositif, caractérisé en ce que les moyens de traitement du signal calculent :

- le logarithme népérien de l'enveloppe du signal reçu ;
- la régression linéaire R du logarithme de l'enveloppe du signal reçu pour les diverses parties de cette enveloppe;

et déterminent la fenêtre temporelle de l'enveloppe correspondant au maximum du coefficient de régression linéaire du logarithme de l'enveloppe et en ce que les moyens de traitement du signal déterminent l'absorption des ondes ultrasonores uniquement dans ladite fenêtre temporelle.

L'invention a également pour objet un dispositif, caractérisé en ce que la sonde comporte des moyens de marquage des corps dont la texture est mesurée et/ou caractérisée, ou d'un coupleur acoustique apposé de façon permanente sur ce corps.

L'invention a également pour objet un procédé de mesure et/ou de caractérisation de la texture, notamment de la dureté d'un corps, caractérisé en ce qu'il comporte les étapes consistant à:

- engendrer dans un corps des vibrations ultrasonores;
- capter les ondes ultrasonores émises par le corps ;
- déterminer l'atténuation des ondes ultrasonores en fonction du temps de réception et de la profondeur de pénétration dans le corps ;
- déduire de l'atténuation des ondes ultrasonores en fonction du temps dans le corps la texture du corps, notamment la dureté du corps.

L'invention a également pour objet un procédé, caractérisé en ce qu'il comporte une étape de détermination de la température du corps dont on mesure ou caractérise la texture et en ce que la détermination de la texture, notamment de la dureté à partir de l'atténuation des ondes ultrasonores dans le corps, tient compte de la température dudit corps.

L'invention a également pour objet un procédé, caractérisé en ce qu'il comporte des étapes consistant à :

- engendrer une succession de vibrations ultrasonores dans le corps dont on veut mesurer ou caractériser la texture ;
- capter les signaux ultrasonores émis par le corps ;
- calculer une succession d'enveloppes des signaux reçus;
- calculer une enveloppe moyenne des enveloppes reçues ;
- déterminer l'atténuation des ondes ultrasonores dans ledit corps à partir de ladite enveloppe moyenne.

L'invention a également pour objet un procédé, caractérisé en ce qu'il comporte une étape de sélection d'une fenêtre temporelle dans les signaux captés par la sonde ayant le meilleur rapport signal/bruit.

L'invention a également pour objet un procédé, caractérisé en ce qu'il comporte les étapes consistant à :

- calculer le logarithme népérien de l'enveloppe du signal reçu;
- calculer la régression linéaire du logarithme népérien de l'enveloppe du signal reçu pour diverses parties de cette enveloppe;
- déterminer la fenêtre temporelle de l'enveloppe correspondant au maximum du coefficient de régression linéaire du logarithme népérien et de l'enveloppe;
- déterminer l'absorption des ondes ultrasonores par le corps uniquement à partir des signaux reçus dans ladite fenêtre temporelle.

L'invention a également pour objet un procédé de mesure et/ou de caractérisation de la texture d'un corps, caractérisé en ce qu'il comporte les étapes consistant à:

- engendrer une succession de vibrations ultrasonores dans le corps dont on veut mesurer ou caractériser la texture ;
- capter les signaux ultrasonores émis par le corps ;
- calculer une succession d'enveloppes des signaux reçus ;
- calculer une enveloppe moyenne des enveloppes reçues ;
- calculer le logarithme népérien de l'enveloppe du signal reçu;
- calculer la régression linéaire du logarithme népérien de l'enveloppe du signal reçu pour diverses parties de cette enveloppe;
- déduire et quantifier la présence de trous, ouvertures et/ou hétérogénéités dans ledit corps à partir de la valeur du coefficient de régression.

L'invention a également pour objet un procédé, caractérisé en ce que ledit procédé est un procédé de mesure ou de caractérisation de la texture d'un produit alimentaire, notamment d'un fromage.

L'invention a également pour objet un procédé, caractérisé en ce qu'il comporte une étape de marquage avec la sonde du corps dont la texture a été mesurée ou caractérisée ou d'un coupleur acoustique apposé de façon permanente sur ce corps, notamment par une empreinte des moyens de marquage de la sonde.

L'invention sera mieux comprise au moyen de la description ci-après et des figures annexées données comme des exemples non limitatifs et sur lesquelles :

- la figure 1 est une courbe expérimentale montrant la corrélation entre l'atténuation des ultrasons et les modules de Young de divers corps ;
- la figure 2 est un schéma d'un premier exemple de réalisation d'un dispositif selon la présente invention ;
- la figure 3 est un schéma d'une sonde susceptible d'être mise en oeuvre dans le dispositif selon la présente invention ;
- la figure 4 est un schéma d'un second exemple de réalisation d'un dispositif selon la présente invention ;
- la figure 5 est une courbe expérimentale d'un signal capté par le dispositif selon la présente invention ;
- la figure 6 est une courbe d'une enveloppe obtenue par calcul à partir de la courbe du signal de la figure 5 ;
- la figure 7 est un schéma d'un premier exemple de réalisation d'un dispositif d'élaboration d'enveloppe susceptible d'être mis en oeuvre dans le dispositif selon la présente invention ;
- la figure 8 est un schéma d'un deuxième exemple de réalisation d'un dispositif d'élaboration d'enveloppe susceptible d'être mis en oeuvre dans le dispositif selon la présente invention ;
- la figure 9 est un schéma illustrant la sommation d'une pluralité d'enveloppes ;
- la figure 10 est une courbe très schématique illustrant le principe d'élimination de bruit permettant d'améliorer le rapport signal/bruit du dispositif selon la présente invention ;
- la figure 11 est une courbe expérimentale illustrant le traitement de réduction du bruit dans le dispositif selon la présente invention ;
- la figure 12 est une courbe illustrant la réduction du bruit dans le dispositif selon la présente invention ;
- les figures 13 à 16 illustrent les traitements mathématiques du signal capté ;
- la figure 17 est un organigramme d'un programme mis en oeuvre dans le dispositif selon la présente invention ;
- la figure 18 est une vue en perspective illustrant un test de qualité final selon la présente invention.

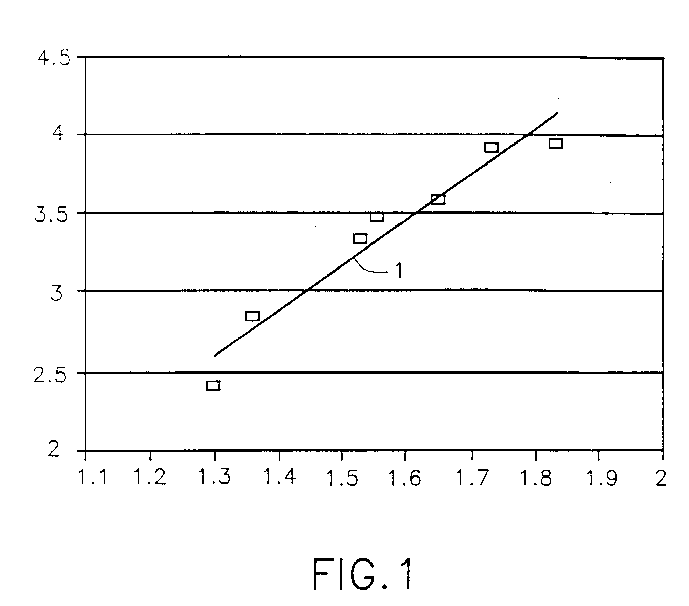

Sur les figures 1 à 18, on a utilisé les mêmes références pour désigner les mêmes éléments.

Lors de tests de laboratoire, la Demanderesse a découvert qu'il existait une corrélation entre la dureté d'un produit et l'atténuation des ultrasons dans ce produit. Sur la figure 1, on a représenté en abscisse l'atténuation des ultrasons exprimés en dB/µs, et en ordonné le module de Young exprimé en N/mm.

Les rectangles représentent des points de mesure expérimentaux correspondant à divers produits à partir desquels, par traitement statistique, on a élaboré une droite 1 de la fonction y = f(x) = Ax + B de la dureté modélisée en fonction de l'atténuation. Les valeurs expérimentales : A égal à 2,85, B égal à - 1,10 et l'écart type quadratique par rapport à la droite égal à 0,94, correspondent à des données hautement corrélées.

La mesure de l'atténuation des ultrasons dans un corps et la connaissance de la loi de corrélation correspondant à la droite 1 permettent d'en déduire la dureté.

Dans le tableau I, on a représenté dans les deux premières colonnes les valeurs expérimentales qui ont été reportées sur la courbe de la figure 1. Dans la troisième colonne, on a représenté la dureté modélisée à partir de la droite 1 donnant une excellente approximation de la dureté réelle. Toutefois, il est bien entendu qu'une modélisation plus précise ne sorte pas du cadre de la présente invention.

TABLEAU I

| Atténuation dB/micro-s | Dureté mesurée N/mm | Dureté modèlisée N/mm |
|---|---|---|
| 1.83 | 3.96 | 4.13 |
| 1.74 | 3.93 | 3.86 |
| 1.65 | 3.60 | 3.60 |
| 1.55 | 3.48 | 3.34 |
| 1.53 | 3.35 | 3.27 |
| 1.37 | 2.88 | 2.81 |
| 1.30 | 2.41 | 2.61 |

De même, lors de tests de laboratoire, la Demanderesse a découvert que l'énergie ainsi que l'amplitude en fonction du temps des ondes ultrasonores rayonnées par un produit insonifié avec des ondes ultrasonores (c'est-à-dire dans lequel l'on a engendré une vibration aux fréquences des ultrasons), en mode de vibration longitudinale sont affectées de façon très importante par la texture du produit insonifié. Il en est de même du spectre de l'énergie rayonnée par le produit. Dans les mesures résumées dans le tableau II, l'on peut voir une très forte corrélation entre la détection de texture par la méthode, selon la présente invention, consistant à analyser l'énergie ultrasonore rayonnée par un produit insonifié avec les ondes ultrasonores longitudinales et les méthodes classiques de surveillance de production.

TABLEAU DES COEFFICIENTS DE CORRELATION ENTRE PARAMETRES:

TABLEAU II

|  | ND | ATT | DUR | COHE | COLL | ELAS | MAST | PRG | G/S | MG |
|---|---|---|---|---|---|---|---|---|---|---|
| ND | 1 | _.93_ | .74 | .85 | .94 | .74 | .95 | .71 | - | - |
| ATT | _.93_ | 1 | .70 | - | - | - | .97 | - | - | - |
| DUR | .74 | .70 | 1 | - | - | - | - | .76 | - | - |
| COHE | .85 | - | - | 1 | - | - | - | - | - | - |
| COLL | .94 | - | - | - | 1 | - | - | - | - | - |
| ELAS | .74 | - | - | - | - | 1 | - | - | - | - |
| MAST | .95 | .97 | - | - | - | - | 1 | - | - | - |
| PRG | .71 | - | .76 | - | - | - | - | 1 | .25 | .65 |
| G/S | - | - | - | - | - | - | - | .25 | 1 | - |

TABLEAU II   (suite)

|  | ND | ATT | DUR | COHE | COLL | ELAS | MAST | PRG | G/S | MG |
|---|---|---|---|---|---|---|---|---|---|---|
| MG | - | - | - | - | - | - | - | .65 | - | 1 |

| ND = Note Dosage (Humaine, visuelle) Note de texture |
|---|
| ATT = Atténuation des Ultra-Sons |
| DUR = Dureté INSTRON |
| COHE = Cohésion INSTRON |
| COLL = Collant INSTRON |
| ELAS = Elasticité INSTRON |
| MAST = Masticabilité INSTRON |
| PRG = P R G (Test pénétration) |
| G/S = Gras/Sec |
| MG = Matières Grasses. |
| Test ISOTKON = test double cycle en compression uniaxiable à vitesse constante. |

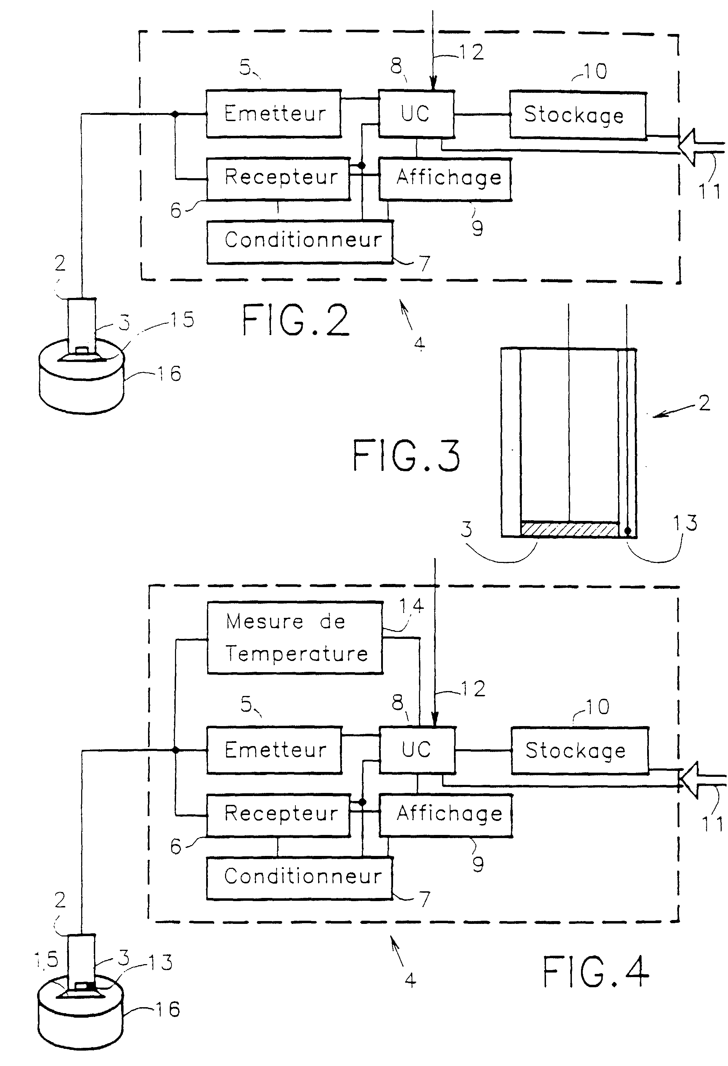

Sur la figure 2, on peut voir un exemple de réalisation d'un dispositif selon la présente invention comportant une sonde 2 comprenant un transducteur électroacoustique 3 et une unité 4 de génération de traitement du signal. L'unité 4 comporte un émetteur d'ultrasons 5, un récepteur d'ultrasons 6, le circuit de conditionnement du signal 7, une unité centrale 8, un dispositif d'affichage 9 et avantageusement un dispositif de stockage des données 10 ainsi qu'une liaison de communication 11. Les éléments 2 à 11 sont avantageusement des dispositifs disponibles dans le commerce. La sonde 2 est avantageusement une sonde à transducteur 3 piézoélectrique servant à l'émission et à la réception de type plane mono-élément, du type décrit dans le Brevet français 87 07795 précité, ou comportant une pluralité de transducteurs piézoélectriques alignés ou formant un secteur permettant la focalisation du faisceau. La fréquence nominale de la sonde est choisie en fonction de la structure du produit étudié. Le transducteur 3 engendre des vibrations ultrasonores longitudinales dans le milieu à étudier.

Il est bien entendu que l'invention n'est pas limitée à l'utilisation d'une même sonde 2 à l'émission et à la réception.

L'émetteur 5 et le récepteur 6 peuvent être regroupés dans un émetteur-récepteur d'ultrasons du commerce, l'émetteur générant par exemple des impulsions de ± 100 V ou plus sous la forme d'impulsions de durées quasi nulles (Dirac), d'impulsions ou de train d'ondes. La fréquence d'échantillonnage de récepteur est supérieure ou égale à 5 fois la fréquence nominale de la sonde.

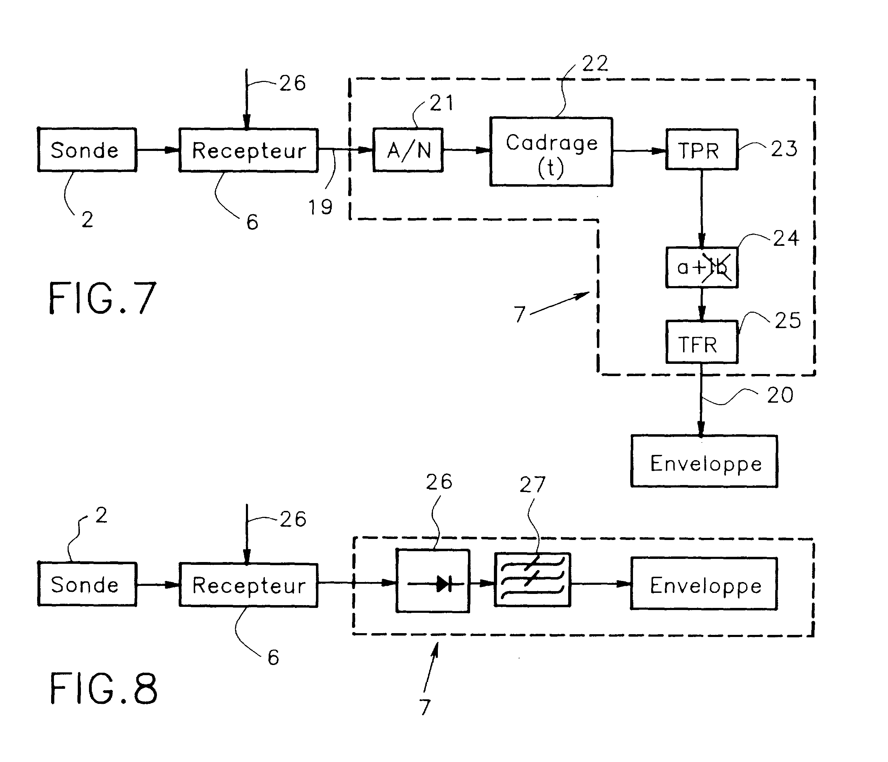

Deux exemples des circuits de conditionnement du signal 7 sont illustrés sur les figures 7 et 8. L'unité centrale 8 est un micro-ordinateur réalisé par exemple sous forme d'une carte VME travaillant par exemple sous système d'exploitation UNIX (marque déposée), un ordinateur personnel fonctionnant sur le système d'exploitation MS-DOS (marque déposée) ou un automate industriel. L'automate industriel peut être disposé à proximité de la sonde 2 sur la chaîne de production dans un environnement relativement hostile. L'on peut aussi bien mettre en oeuvre un automate dédié que relier la sonde 2 à un automate déjà présent sur la chaîne de production et assurant d'autres tâches.

Lors d'essais d'installation, la Demanderesse a découvert que, contrairement à l'opinion généralement admise, les signaux électriques radiofréquences destinés à engendrer les ultrasons ou générés par la capture des ultrasons par la sonde 2 peuvent être transmis par un câble sur des distances pouvant atteindre et même dépasser une centaine de mètres. On a, par exemple, utilisé avec succès un câble coaxial blindé (3 conducteurs coaxiaux) d'une centaine de mètres pour relier la sonde 2 à l'unité 4. Dans un tel cas, l'on peut disposer l'unité 4 dans un endroit protégé, par exemple dans un local, ce qui permet de mettre en oeuvre un ordinateur de bureau qui ne supporterait pas l'environnement hostile d'une chaîne de production. Avantageusement, l'on met en oeuvre une sonde étanche susceptible de fonctionner dans un environnement hostile et d'être nettoyée facilement et complètement pour préserver l'hygiène de la chaîne de production.

De même, il est possible de séparer l'émetteur et le récepteur de l'unité centrale en les reliant par un câble. La liaison peut être réalisée aussi bien en analogique qu'en numérique et peut éventuellement être multiplexée.

Dans un premier exemple de réalisation, le dispositif d'affichage 9 comportent uniquement des afficheurs numériques ou alphanumériques indiquant les valeurs mesurées des étapes de cycle de production et/ou des alarmes. Les alarmes visuelles peuvent éventuellement être complétées par des alarmes sonores. On utilise par exemple des afficheurs à cristaux liquides, à diodes électro-luminescentes ou un tube à rayons cathodiques associé à un circuit de commande.

Dans une variante de réalisation, le dispositif d'affichage 9 comporte un écran graphique associé à une carte d'affichage.

L'unité de stockage de données 10 comporte, par exemple, un disque dur, un lecteur de disquettes ou des mémoires électroniques permanentes de type mémoires dynamiques (RAM en terminologie anglo-saxonne) secourues, des mémoires mortes programmables effaçables électriquement (EEPROM en terminologie anglo-saxonne) ou des mémoires à bulles.

Avantageusement, l'on stocke les données de mesure sous un format standard de tableur ou de base de données pour un traitement ultérieur.

La liaison de communication 11 comporte, par exemple, un port série RS-232.

L'unité centrale 8 est reliée à tous les autres éléments de l'unité 4 de génération et de traitement de signal. Elle reçoit, d'autre part, un signal de commande externe 12.

L'émetteur 5 et le récepteur 6 sont reliés à la sonde 2. Le récepteur est relié au circuit de conditionnement du signal 7 et au dispositif d'affichage 9. Le circuit de conditionnement du signal 7 est relié au dispositif d'affichage 9. Le dispositif de stockage 10 est avantageusement relié par un accès direct mémoire (DMA en terminologie anglo-saxonne) à la liaison de communication 11.

Une variation de température peut fausser les résultats de mesure de texture selon l'invention en modifiant la correspondance biunivoque entre les valeurs de la dureté et les valeurs de l'atténuation des ultrasons. Pour des mesures qui devraient être effectuées dans une plage de température restreinte, un étalonnage initial du dispositif selon l'invention s'avère suffisant.

Par contre, pour pouvoir effectuer des mesures dans une large gamme de température, il s'avère avantageux de stocker dans l'unité centrale 12 ou dans l'unité de stockage 10 une table de correspondance entre la dureté et l'atténuation des ultrasons pour chaque plage des températures. Les largeurs des plages de températures assignées aux diverses tables et donc le nombre de tables dépendent de la précision désirée.

Le choix de la table à appliquer est soit effectué manuellement par l'opérateur qui communique, par l'intermédiaire d'un clavier ou d'un commutateur, la température du corps dont on veut mesurer ou caractériser la texture ou la dureté, soit, avantageusement, automatiquement par des moyens d'acquisition de la température connectés à l'unité centrale 8 de l'unité 4 de gestion et de traitement du signal. Sur la figure 3, l'on peut voir un exemple de sonde 2 comportant un dispositif de mesure de températures, par exemple un thermocouple 13. Dans l'exemple illustré sur la figure 3, le point sensible, c'est-à-dire la soudure chaude du thermocouple, est situé à proximité du transducteur 3 sur la face avant de la sonde 2. Avantageusement, la paroi de la sonde 2 est réalisée en acier inoxydable.

La sonde 2 de la figure 3 est reliée à une unité 4 de génération et de traitement du signal de la figure 4 par un premier ensemble de conducteurs électriques véhiculant les impulsions à émettre et/ou les signaux reçus et un second ensemble de conducteurs véhiculant un signal comportant une information sur la température du corps dont on veut connaître la texture vers un dispositif de mesure de température 14 (de type connu) de l'unité 4 de génération et de traitement du signal de la figure 4. Avantageusement, les conducteurs reliés à l'émetteur 5 et au récepteur 6 d'une part, et ceux reliés au dispositif de mesure de température 14 sont regroupés dans un câble unique.

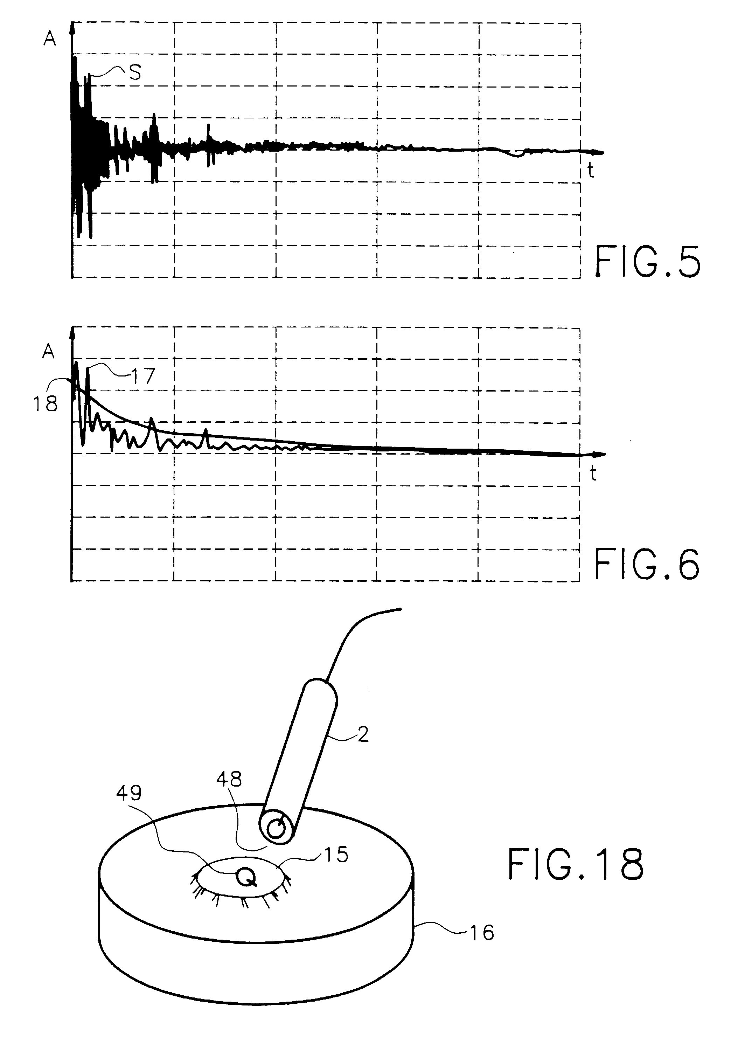

Sur les figures 5, 6 et 9 à 16, on a porté le temps en abscisse et l'amplitude du signal reçu en ordonné.

Lors d'une mesure ou de la caractérisation de la texture, l'émetteur 5 génère une impulsion moyenne tension qui arrive au transducteur 3 de la sonde 2 qui engendre une impulsion de vibrations mécaniques du transducteur 3 à des fréquences ultrasonores, par exemple comprises entre 0,05 et 50 MHz. Les vibrations mécaniques se propagent, éventuellement à travers un coupleur acoustique 15 dans un corps 16 dont on veut caractériser ou mesurer la texture, par exemple dans un fromage en cours de fabrication ou à la fin de celle-ci. Lorsqu'on envoie une impulsion mécanique de durée $\tau$, la sonde capte un signal S haute fréquence dont l'amplitude varie avec le temps tel qu'illustré sur la figure 5. Ce signal est converti par le transducteur 3 de la sonde 2 en un signal électrique de même allure qui est transmis au récepteur 6. La partie intéressante du signal correspondant à un décalage temporel par rapport à l'émission est transmise par le récepteur 6 au circuit de conditionnement du signal 7 qui en extrait une enveloppe 17, telle qu'illustrée sur la figure 6. D'autre part, on a représenté sur la figure 6 une courbe 18 de l'atténuation qui est un résultat intermédiaire de traitement destiné à déterminer la dureté et/ou la texture du produit.

Un premier exemple de circuit 7 de conditionnement du signal est illustré sur la figure 7. Ce circuit 7 comporte connecté en série entre une entrée 19 et une sortie 20, un convertisseur analogique/numérique 21, un circuit de cadrage temporel 22, un circuit de calcul de la transformée de Fourier rapide 23 (FFT en terminologie anglo-saxonne), un circuit 24 d'élimination de composants imaginaires du spectre de puissance et un circuit de calcul de la transformée de Fourier rapide inverse (FFT$^{-1}$ en terminologie anglo-saxonne).

L'entrée 19 du circuit 7 de conditionnement du signal est connectée à la sortie du récepteur 6. Le convertisseur analogique/numérique 21 assure la numérisation du signal reçu. Le dispositif de cadrage temporel 22 envoie un signal de commande 26 au récepteur 6 déterminant le début et la fin de la transmission des signaux reçus vers les circuits 7. Les circuits de calcul de la transformée de Fourier rapide 23 effectuent les calculs de la transformée de Fourier discrète (par l'algorithme de calcul de la transformée de Fourier rapide) et délivrent un spectre de puissance du signal au circuit 24. Le circuit 24 effectue un filtrage numérique éliminant les composantes imaginaires du spectre de puissance. Le circuit de calcul de la transformée de Fourier inverse 25 effectue le calcul de transformée de Fourier discrète inverse délivrant une enveloppe temporelle 17 de la figure 6.

Sur la figure 8, on peut voir un second exemple de réalisation d'un circuit 7 de conditionnement du signal comportant connecté en série un circuit 26 redresseur du signal et un filtre passe-bas 27.

Le circuit redresseur de signal, connecté à la sortie du récepteur 6, assure l'élimination des signaux correspondant à une amplitude négative. Le filtre passe-bas 27 assure l'élimination des hautes et moyennes fréquences.

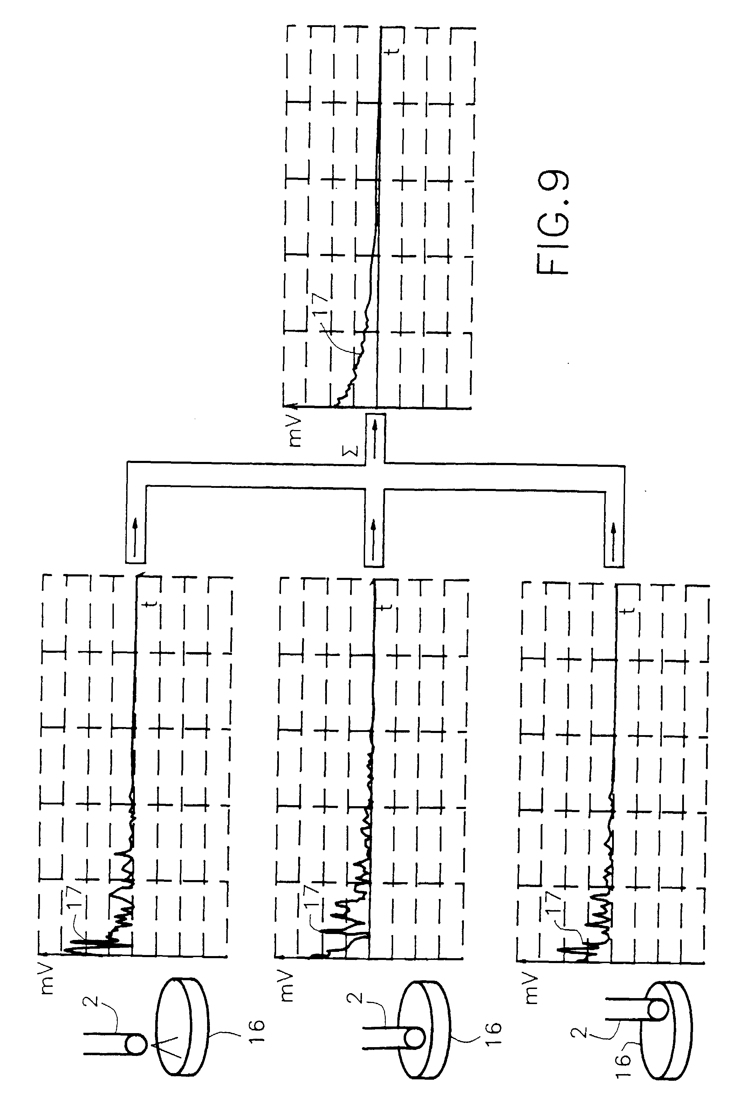

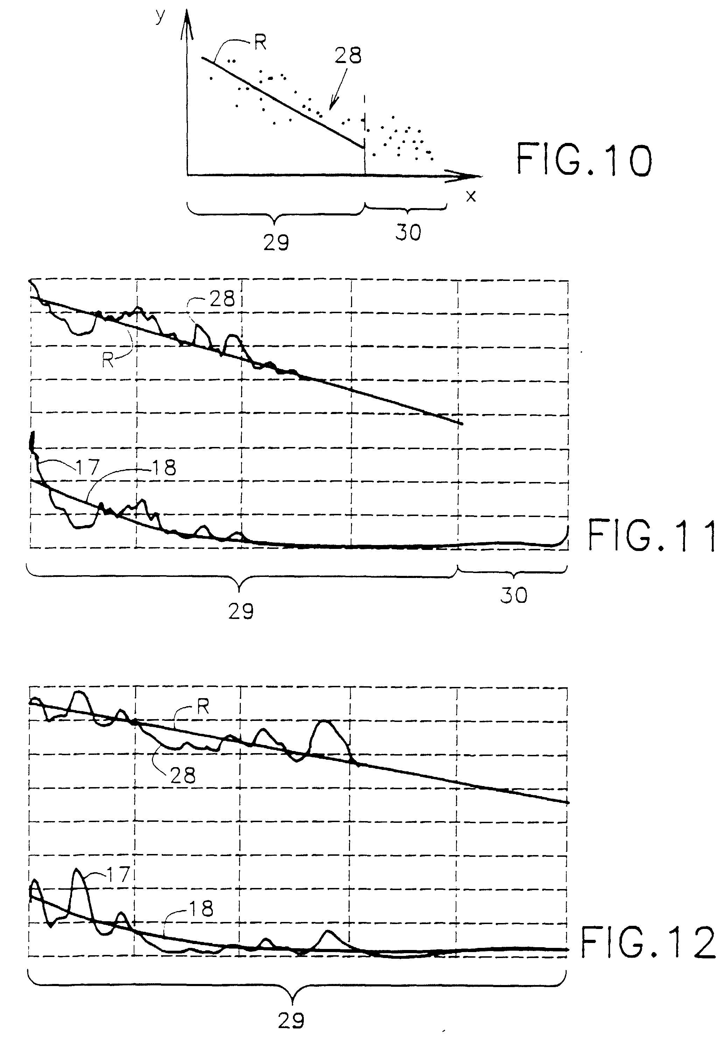

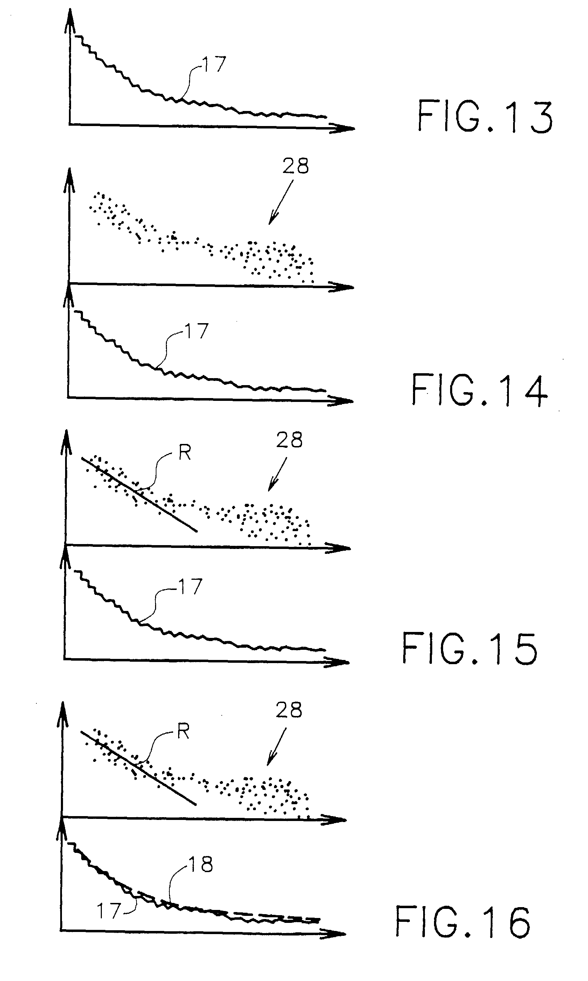

Sur la figure 9, on peut voir le calcul d'une enveloppe moyenne, sur la figure 10 le principe de l'élimination du bruit selon l'invention, sur les figures 11 et 12 des résultats de mesures réels et sur les figures 13 à 16 les diverses étapes de traitement selon la présente invention.

Sur la figure 9, on peut voir l'étape de calcul de la moyenne des enveloppes du signal reçu. La sonde 2 est placée dans une première position par rapport à un fromage 16 dont on veut mesurer ou caractériser la texture. Après émission d'une impulsion ultrasonore, on recueille un signal rétro-diffusé qui, traité par le circuit conditionneur 7, devient une première enveloppe 17. On répète la mesure pour une position différente de la sonde 2 par rapport au fromage 16 et ainsi de suite jusqu'à l'obtention du nombre d'enveloppes 17 désiré, par exemple jusqu'à l'obtention de 100 enveloppes. On calcule la moyenne de toutes les valeurs 17, pour obtenir une enveloppe moyenne utilisée pour la suite du traitement.

Les principes de l'amélioration du rapport signal/bruit sont illustrés sur les figures 13 à 16.

D'une part, une mesure fiable et fidèle nécessite la mise en oeuvre d'un dispositif ayant un bon rapport signal/bruit.

D'autre part, l'utilisation en milieu industriel suppose un traitement automatique de l'information sans que l'opérateur, typiquement un technicien sans qualification particulière en traitement du signal ou en instrumentation, n'ait à calibrer l'appareil pour chaque mesure ou série de mesures.

Avantageusement, le dispositif selon la présente invention effectue un cadrage temporel de l'intervalle significatif de l'enveloppe 17 de la figure 6 en éliminant les parties de cette courbe qui représentent un bruit acoustique et/ou électrique important, ce qui permet d'effectuer un calcul précis et fiable de l'atténuation des ultrasons.

Sur la figure 10, on peut voir le logarithme népérien 28 de l'enveloppe moyenne 17 de la figure 9. Dans la mesure où l'enveloppe 17 est une exponentielle décroissante, son logarithme 28 devrait être une droite. Toutefois, il résulte du bruit de mesure, de quantification et de calcul que le logarithme népérien 28 est un nuage de points.

En se référant à la figure 10, l'on calcule la régression linéaire R des divers intervalles de points du nuage de points 28 du logarithme de l'enveloppe 17. On valide une fenêtre de calcul 29 ayant le nombre de points correspondant à une régression linéaire maximale et l'on élimine les points se trouvant dans l'intervalle 30 présentant un mauvais rapport signal/bruit.

Le cas illustré sur la figure 11 correspond aux valeurs suivantes :

- gain récepteur : 41dB,
- gain de l'affichage : 41dB,
- fréquence d'échantillonnage : 50 MHz,
- délai d'affichage : 9,8 µs,
- gain de l'affichage : 0,1 V/division,
- déplacement de l'affichage : 0,04 division,
- point de départ de la ligne : 1,
- points de la ligne : 2048,
- points de l'enveloppe : 2048,
- incrément dans le calcul de régression linéaire R du logarithme 28 de l'enveloppe 17 : 10.

Résultats des calculs obtenus :

- points de la fenêtre de calcul 29 : 391,
- coefficient de régression : 97,340 %,
- pente de la régression : 2,7 dB/µs.

La courbe 17 de la partie inférieure de la figure 11 correspond à la moyenne des enveloppes mesurées. Le logarithme 28 de l'enveloppe 17 et sa régression linéaire maximale R correspondant à la fenêtre 29 retenue sont affichés dans la partie supérieure de la figure 11. On en déduit une enveloppe modélisée 18 correspondant à une exponentielle décroissante dont le coefficient a été calculé.

Le cas illustré sur la figure 12 correspond aux valeurs suivantes :

- gain récepteur: 46,4 dB,
- gain d'affichage : 46,4 dB,
- fréquence d'échantillonnage : 100 MHz,
- délais d'affichage : 9,2 µs,
- gains d'affichage : 0,1 V/division,

- déplacement de l'affichage: 0,04 division,
- point de départ de la ligne : 1,
- points de l'enveloppe : 2001,
- incrément dans le calcul de régression linéaire : 10.

Résultats des calculs obtenus :

- point de la régression : 11,
- coefficient de la régression : 95,617 %,
- pente de la régression : 3,6 dB/µs.

L'enveloppe moyenne 17 de la figure 9 est illustrée sur la figure 13. L'on calcule le logarithme 28 de l'enveloppe moyenne 17 tel qu'illustré sur la figure 14.

Sur la figure 15, l'on calcule la régression linéaire R du logarithme 28. La régression linéaire est de la forme $y = mx + b$, m étant la pente de la régression linéaire, c'est-à-dire l'atténuation des ondes ultrasonores et b étant l'ordonné à l'origine, c'est-à-dire le niveau de l'énergie diffusée. Pour un nuage de N points,

$$m = \frac{\Sigma x_i y_i - (\Sigma x_i \, \Sigma y_i)/N}{\Sigma x_i^2 - \dfrac{(\Sigma x_i)^2}{N}}$$

$$\sigma x = \left[\frac{\Sigma x_i^2 - \dfrac{(\Sigma x_i)^2}{N}}{N-1}\right]^{1/2}$$

$$\sigma y = \left[\frac{\Sigma y_i^2 - \dfrac{(\Sigma y_i)^2}{N}}{N-1}\right]^{1/2}$$

$$R = m \, \frac{\sigma x}{\sigma y}$$

R étant le coefficient de la régression linéaire.

Dans l'exemple de réalisation avantageux, l'on calcule tout d'abord la régression linéaire sur N1 points, N1 étant par exemple égal à N/10. L'on calcule le coefficient de régression $R_1$ pour ce premier ensemble des points. L'on ajoute un incrément de points à l'ensemble des points sur lequel on effectue le calcul, cet incrément étant par exemple égal à N/10 et l'on calcule un second coefficient de régression linéaire $R_2$. L'on ajoute de nouveau un incrément de points à l'ensemble des points traités et l'on recalcule un nouveau coefficient de régression linéaire $R_3$ et ainsi de suite jusqu'à ce que l'on ait atteint les N points mesurés. Le nombre de points pour lequel le coefficient de régression linéaire est maximal donne le nombre de points utiles de la moyenne de l'enveloppe et le coefficient de régression linéaire de cet ensemble de points donne l'atténuation. Il est à noter que la présence de trous dans un fromage entraîne une perte de précisions dans la mesure de l'atténuation. La sélection du maximum du coefficient de régression linéaire permet d'obtenir des mesures acceptables. Toutefois, la valeur maximale du coefficient de régression linéaire dans le cas d'un fromage comportant des trous sera plus faible, par exemple égale à 94 %. La valeur du coefficient de régression linéaire, au-dessus de la valeur minimale est le reflet des hétérogénéités de la pâte pour une longueur d'ondes donnée de la sonde à ultrasons.

Sur la figure 16, on a représenté une enveloppe modélisée 18 qui est une exponentielle de croissance dont le coefficient est proportionnel à l'absorption modélisée.

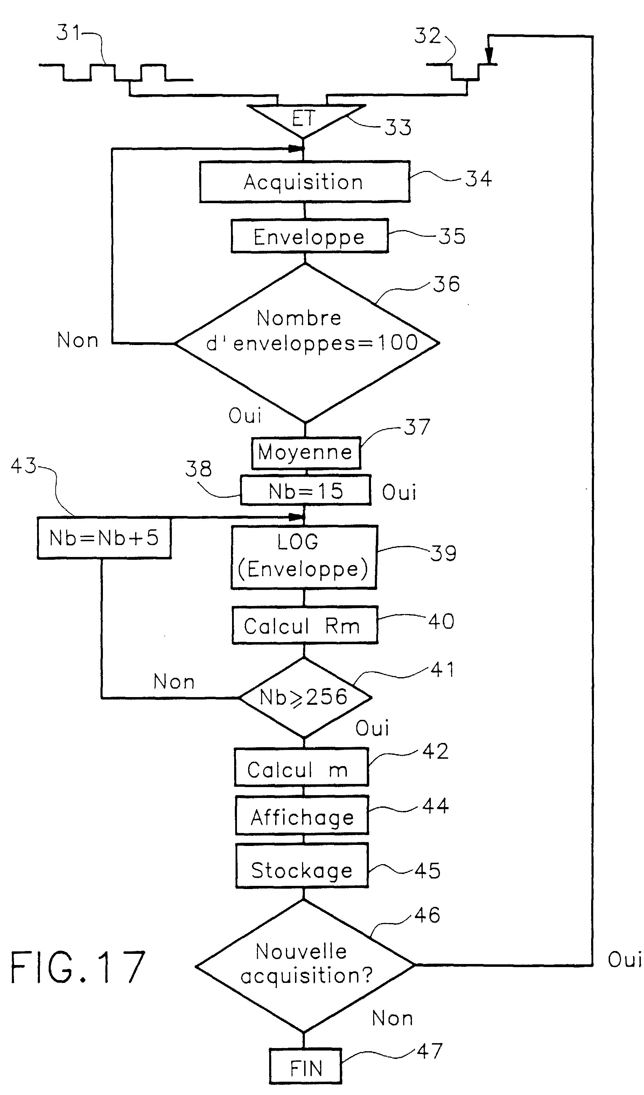

Sur la figure 17, on peut voir un organigramme d'un exemple de programme mis en oeuvre par une unité de génération et de traitement du signal 4 des dispositifs des figures 1 et 3. L'unité centrale 8 reçoit en permanence un signal d'horloge d'échantillonnage 31 fournissant la fréquence d'échantillonnage du signal analogique. L'unité centrale 8 reçoit, d'autre part, des impulsions de déclenchement de la mesure 32. Lors de la présence simultanée du signal 31 et du signal 32, la porte ET 33 déclenche l'acquisition au début de la première période d'horloge d'échantillonnage 31 suivant la présence de l'impulsion 32 de déclenchement de l'acquisition.

On va en 34.

En 34, l'on effectue l'acquisition d'une ligne d'échographie, c'est-à-dire que l'émetteur 5 envoie une impulsion électrique à la sonde 2 et que le récepteur 6 reçoit de la sonde 2, un signal S tel que par exemple illustré sur la figure 5. Le signal S est numérisé.

On va en 35.

En 35, l'on effectue le calcul de l'enveloppe 17 du signal.

On va en 36.

En 36, l'on vérifie si le nombre d'enveloppes calculé est égal à 100.

Si non, on va en 34.

Si oui, on va en 37.

En 37, l'on effectue le calcul de la moyenne des enveloppes reçues donnant une enveloppe moyenne telle qu'illustrée par exemple sur la figure 9.

On va en 38.

En 38, l'on initialise, par exemple à 15, le compteur Nb de points de l'enveloppe.

On va en 39.

En 39, l'on calcule le logarithme népérien des Nb premiers points de l'enveloppe moyenne.

On va en 40.

En 40, l'on calcule la régression linéaire, la pente et le coefficient de régression. On stocke en mémoire la valeur maximale du coefficient de régression ainsi que la valeur du compteur Nb correspondante.

On va en 41.

En 41, l'on vérifie si la valeur du compteur Nb des enveloppes est supérieure ou égale au nombre de points de la ligne d'échographie, par exemple à 256.

Si oui, on va en 42.

Si non, on va en 43.

En 43, l'on incrémente le compteur Nb du nombre de points de l'enveloppe d'une valeur, par exemple égale à 5.

On va en 39.

En 42, l'on effectue le calcul de l'atténuation m des ultrasons qui est la pente de la droite de régression pour le nombre de points qui donne le plus grand coefficient de régression.

On va en 44.

En 44, l'on affiche l'atténuation et le coefficient de régression. Cet affichage est adapté au type de mesure effectuée. Par exemple, on utilisera un affichage numérique et/ou graphique dans un laboratoire donnant la courbe complète, alors que sur une chaîne de production, on affichera directement si la qualité du produit fabriqué, par exemple d'un fromage, est bonne ou non. Par exemple, on peut indiquer uniquement par une diode rouge qu'une intervention est nécessaire et par une diode verte que le processus de fabrication se passe correctement. De même, il est possible de remplacer ou de compléter l'affichage de résultat 43 par l'envoi de consignes de commandes par l'unité centrale 8 à des automates de production pour assurer la correction des paramètres du processus mis en jeu.

On va en 45.

En 45, l'on effectue le stockage, par exemple sur un disque dur ou sur des disquettes, de l'atténuation et du coefficient de régression éventuellement complété par des données concernant l'échantillon traité, la date et/ou l'heure.

On va en 46.

Dans le cas d'un processus manuel, en 45, l'on demande à l'opérateur s'il désire une nouvelle acquisition.

Si oui, l'on va en 32 en envoyant une nouvelle impulsion de déclenchement d'acquisition.

Si non, on va en 47.

En 47, l'on sort du programme de traitement du signal.

La mesure d'atténuation des ondes ultrasonores à l'intérieur de certains fromages, notamment des fromages à croûte sèche peut s'avérer difficile sans l'utilisation d'un coupleur acoustique. Selon l'invention, l'on peut utiliser un coupleur acoustique réalisé sous la forme d'une cire, par exemple une cire ayant une impédance acoustique $Z = \rho c$ proche ou inférieure à celle de l'eau qui est égale à 1,5 pour une vitesse de propagation égale à 1500 m/s à 20°C. Une fois appliqué sur le fromage, le coupleur acoustique 15 doit être le plus homogène possible, et notamment ne pas comporter des micro-bulles d'air. Avantageusement, comme on peut le voir sur la figure 18, la sonde 2 comporte des moyens de marquage 48 permettant de marquer par exemple par une empreinte 49 le coupleur acoustique 15 apposé sur le fromage 16 ou la croûte du fromage 16. Ainsi, l'on assure un marquage des fromages dont la qualité a été vérifiée. Ce marquage permet, d'une part, de s'assurer que ce fromage a été vérifié sur la chaîne de production, et d'autre part de fournir aux consommateurs une garantie de la qualité du produit. Les moyens de marquage 48 peuvent correspondre à une marque déposée du fabricant de fromages, à la lettre Q ou à l'inscription de type "contrôle de qualité". Il est bien évident que les fromages ayant échoués au contrôle de qualité sont retirés de la commercialisation, ou, tout au moins, l'on procède à l'ablation du coupleur acoustique 15 portant l'empreinte 49 des moyens de marquage 48.

L'invention s'applique à toute industrie nécessitant la caractérisation ou la mesure de texture, et notamment à l'industrie alimentaire, pharmaceutique, cosmétologique, plastique et aux industries de peinture.
L'invention s'applique principalement à la fabrication de fromages.

**Revendications**

1. Dispositif de mesure et/ou de caractérisation de la texture d'un corps (16) caractérisé en ce qu'il comporte une sonde (2) d'émission et de réception d'ondes ultrasonores, reliée à un émetteur (5) pour générer un signal électrique converti en ondes ultrasonores par la sonde (2) et un récepteur (6) des ondes ultrasonores émanant dudit corps (16) et captées par la sonde (2) ainsi que des moyens de traitement du signal (7,8) pour déterminer l'atténuation dans ledit corps (16) des ondes ultrasonores en fonction du temps de réception et donc de la profondeur de pénétration dans le corps (16) et pour en déduire la texture, notamment la dureté dudit corps (16).

2. Dispositif selon la revendication 1, caractérisé en ce que la sonde (2) comporte, en outre, des moyens (13, 14) de mesure de la température du corps (16) et en ce que les moyens de traitement du signal (7,8) tiennent compte de la température du corps (16) lors de la détermination de la texture à partir des résultats de la mesure d'absorption des ondes ultrasonores.

3. Dispositif selon la revendication 1 ou 2, caractérisé en ce que les moyens de traitement du signal (7,8) calculent à partir d'une succession de signaux ultrasonores captés par la sonde (2), une succession d'enveloppes des signaux reçus dudit corps pour en déduire une enveloppe moyenne à partir de laquelle on détermine l'atténuation des ondes ultrasonores dans ledit corps.

4. Dispositif selon l'une quelconque des revendications précédentes, caractérisé en ce que le dispositif de traitement du signal (7,8) sélectionne automatiquement une fenêtre temporelle (29) dans le signal capté par la sonde (2) ayant le meilleur rapport signal/bruit.

5. Dispositif selon la revendication 4, caractérisé en ce que les moyens de traitement du signal (7,8) calculent :

   - le logarithme népérien de l'enveloppe du signal reçu ;
   - la régression linéaire R du logarithme (28) de l'enveloppe (17) du signal reçu pour les diverses parties de cette enveloppe ;

   et déterminent la fenêtre temporelle (29) de l'enveloppe (17) correspondant au maximum du coefficient de régression linéaire (R) du logarithme (28) de l'enveloppe et en ce que les moyens de traitement du signal (7,8) déterminent l'absorption des ondes ultrasonores uniquement dans ladite fenêtre temporelle (29).

6. Dispositif selon l'une quelconque des revendications précédentes, caractérisé en ce que la sonde (2) comporte des moyens (48) de marquage des corps (16) dont la texture est mesurée et/ou caractérisée, ou d'un coupleur acoustique (15) apposé de façon permanente sur ce corps (16).

7. Procédé de mesure et/ou de caractérisation de la texture, notamment de la dureté d'un corps (16), caractérisé en ce qu'il comporte les étapes consistant à :

   - engendrer dans un corps (16) des vibrations ultrasonores ;
   - capter les ondes ultrasonores émises par le corps (16) ;
   - déterminer l'atténuation des ondes ultrasonores en fonction du temps de réception et de la profondeur de pénétration dans le corps (16) ;
   - déduire de l'atténuation des ondes ultrasonores en fonction du temps dans le corps (16) la texture du corps, notamment la dureté du corps (16).

8. Procédé selon la revendication 7, caractérisé en ce qu'il comporte une étape de détermination de la température du corps (16) dont on mesure ou caractérise la texture et en ce que la détermination de la texture, notamment de la dureté à partir de l'atténuation des ondes ultrasonores dans le corps (16), tient compte de la température dudit corps (16).

9. Procédé selon la revendication 7 ou 8, caractérisé en ce qu'il comporte des étapes consistant à :

- engendrer une succession de vibrations ultrasonores dans le corps (16) dont on veut mesurer ou caractériser la texture ;
- capter les signaux ultrasonores émis par le corps (16) ;
- calculer une succession d'enveloppes (17) des signaux reçus (S) ;
- calculer une enveloppe moyenne des enveloppes reçues (17) ;
- déterminer l'atténuation des ondes ultrasonores dans ledit corps (16) à partir de ladite enveloppe moyenne.

10. Procédé selon l'une quelconque des revendications 7 à 9, caractérisé en ce qu'il comporte une étape de sélection d'une fenêtre temporelle (29) dans les signaux captés (S) par la sonde (2) ayant le meilleur rapport signal/bruit.

11. Procédé selon la revendication 10, caractérisé en ce qu'il comporte les étapes consistant à :

- calculer le logarithme népérien (28) de l'enveloppe du signal reçu ;
- calculer la régression linéaire (R) du logarithme népérien (28) de l'enveloppe (17) du signal (S) reçu pour diverses parties de cette enveloppe ;
- déterminer la fenêtre temporelle (29) de l'enveloppe (17) correspondant au maximum du coefficient de régression linéaire (R) du logarithme népérien (28) et de l'enveloppe ;
- déterminer l'absorption des ondes ultrasonores par le corps (16) uniquement à partir des signaux reçus dans ladite fenêtre temporelle (29).

12. Procédé de mesure et/ou de caractérisation de la texture d'un corps (16) caractérisé en ce qu'il comporte les étapes consistant à :

- engendrer une succession de vibrations ultrasonores dans le corps (16) dont on veut mesurer ou caractériser la texture ;
- capter les signaux ultrasonores émis par le corps (16) ;
- calculer une succession d'enveloppes (17) des signaux reçus (S) ;
- calculer une enveloppe moyenne des enveloppes reçues (17) ;
- calculer le logarithme népérien (28) de l'enveloppe du signal reçu ;
- calculer la régression linéaire (R) du logarithme népérien (28) de l'enveloppe (17) du signal (S) reçu pour diverses parties de cette enveloppe ;
- déduire et quantifier la présence de trous, ouvertures et/ou hétérogénéités dans ledit corps à partir de la valeur du coefficient de régression.

13. Procédé selon l'une quelconque des revendications 7 à 12, caractérisé en ce que ledit procédé est un procédé de mesure ou de caractérisation de la texture d'un produit alimentaire, notamment d'un fromage.

14. Procédé selon l'une quelconque des revendications 7 à 13, caractérisé en ce qu'il comporte une étape de marquage avec la sonde (2) du corps (16) dont la texture a été mesurée ou caractérisée ou d'un coupleur acoustique (15) apposé de façon permanente sur ce corps, notamment par une empreinte (49) des moyens de marquage (48) de la sonde (2).

**Patentansprüche**

1. Einrichtung zur Messung und/oder Charakterisierung der Textur bzw. Struktur eines Körpers (16), dadurch **gekennzeichnet**, daß sie eine Sonde (2) für die Aussendung und den Empfang von Ultraschallwellen hat, die mit einem Sender (5) zum Erzeugen eines elektrischen Signals, das durch die Sonde (2) in Ultraschallwellen umgewandelt wird, und einem Empfänger (6) für Ultraschallwellen, die von dem Körper (16) ausgehen und durch die Sonde (2) eingefangen werden, verbunden ist, sowie Mittel für die Verarbeitung des Signals (7, 8) zum Bestimmen der Dämpfung der Ultraschallwellen in dem Körper (16) in Abhängigkeit von der Zeit des Empfangs und demgemäß der Tiefe des Eindringens in den Körper (16) und zum daraus Ableiten der Textur bzw. Struktur, insbesondere der Härte, des Körpers (16).

2. Einrichtung gemäß Anspruch 1, dadurch **gekennzeichnet**, daß die Sonde (2) außerdem Mittel (13, 14) zur Messung der Temperatur des Körpers (16) hat, und daß die Mittel für die Verarbeitung des Signals (7, 8) die Temperatur des Körpers (16) während der Bestimmung der Textur bzw. Struktur, ausgehend von den Ergebnissen der Messung der Absorption der Ultraschallwellen, berücksichtigen.

**3.** Einrichtung gemäß Anspruch 1 oder 2, dadurch **gekennzeichnet**, daß die Mittel für die Verarbeitung des Signals (7, 8), ausgehend von einer Aufeinanderfolge von Ultraschallsignalen, die durch die Sonde (2) eingefangen worden sind, eine Aufeinanderfolge von Hüllen der von dem Körper empfangenen Signale berechnen, um daraus eine mittlere Hülle abzuleiten, von welcher ausgehend man die Dämpfung der Ultraschallwellen in dem Körper bestimmt.

**4.** Einrichtung gemäß irgendeinem der vorhergehenden Ansprüche, dadurch **gekennzeichnet**, daß die Einrichtung für die Verarbeitung des Signals (7, 8) automatisch ein zeitliches Fenster (29) in dem durch die Sonde (2) eingefangenen Signal auswählt, welches das beste Signal/Rauschen-Verhältnis hat.

**5.** Einrichtung gemäß Anspruch 4, dadurch **gekennzeichnet**, daß die Mittel für die Verarbeitung des Signals (7, 8) folgendes berechnen:

- den neperischen Logarithmus der Hülle des empfangenen Signals;
- die lineare Regression (R) des Logarithmus (28) der Hülle (17) des empfangenen Signals für die verschiedenen Teile dieser Hülle;

und das zeitliche Fenster (29) der Hülle (17) bestimmen, welches dem Maximum des Koeffizienten der linearen Regression (R) des Logarithmus (28) der Hülle entspricht, und daß die Mittel für die Verarbeitung des Signals (7, 8) die Absorption der Ultraschallwellen allein in dem genannten zeitlichen Fenster (29) bestimmen.

**6.** Einrichtung gemäß irgendeinem der vorhergehenden Ansprüche, dadurch **gekennzeichnet**, daß die Sonde (2) Mittel (48) für die Markierung des Körpers (16), dessen Textur bzw. Struktur gemessen und/oder charakterisiert wird, oder eines akustischen Kopplers (15), der in permanenter Art und Weise auf diesen Körper (16) aufgesetzt bzw. aufgeklebt ist, hat.

**7.** Verfahren zur Messung und/oder Charakterisierung der Textur bzw. Struktur, insbesondere der Härte, eines Körpers (16), dadurch **gekennzeichnet**, daß es die folgenden Schritte hat, bestehend in dem:

- Erzeugen von Ultraschallvibrationen bzw. -schwingungen in einem Körper (16);
- Einfangen von Ultraschallwellen, die durch den Körper (16) ausgesandt worden sind;
- Bestimmen der Dämpfung der Ultraschallwellen in Abhängigkeit von der Zeit des Empfangs und der Tiefe des Eindringens in den Körper (16);
- Ableiten der Textur bzw. Struktur des Körpers, insbesondere der Härte des Körpers (16), von der Dämpfung der Ultraschallwellen in Abhängigkeit von der Zeit in dem Körper (16).

**8.** Verfahren gemäß Anspruch 7, dadurch **gekennzeichnet**, daß es einen Schritt der Bestimmung der Tempertur des Körpers (16), von dem man die Textur bzw. Struktur mißt oder charakterisiert, hat, und daß die Bestimmung der Textur bzw. Struktur, insbesondere der Härte, ausgehend von der Dämpfung der Ultraschallwellen in dem Körper (16) die Temperatur dieses Körpers (16) berücksichtigt.

**9.** Verfahren gemäß Anspruch 7 oder 8, dadurch **gekennzeichnet**, daß es Schritte hat, bestehend aus dem:

- Erzeugen einer Aufeinanderfolge von Ultraschallvibrationen bzw. -schwingungen in dem Körper (16), von dem man die Textur bzw. Struktur messen oder charakterisieren will ;
- Einfangen der Ultraschallsignale, die von dem Körper (16) ausgesandt worden sind ;
- Berechnen einer Aufeinanderfolge von Hüllen (17) der empfangenen Signale (S) ;
- Berechnen einer mittleren Hülle der empfangenen Hüllen (17) ;
- Bestimmen der Dämpfung der Ultraschallwellen in dem genannten Körper (16), ausgehend von der genannten mittleren Hülle.

**10.** Verfahren gemäß irgendeinem der Ansprüche 7 bis 9, dadurch **gekennzeichnet**, daß es einen Schritt der Auswahl eines zeitlichen Fensters (29) in den durch die Sonde (2) eingefangenen Signalen (S), welches das beste Signal/Rauschen-Verhältnis hat, beinhaltet.

**11.** Verfahren gemäß Anspruch 10, dadurch **gekennzeichnet**, daß es die Schritte hat, bestehend aus dem:

- Berechnen des neperischen Logarithmus (28) der Hülle des empfangenen Signals;

- Berechnen der linearen Regression (R) des neperischen Logarithmus (28) der Hülle (17) des empfangenen Signals (S) für verschiedene Teile dieser Hülle;
- Bestimmen des zeitlichen Fensters (29) der Hülle (17), welches dem Maximum des Koeffizienten der linearen Regression (R) des neperischen Logarithmus (28) und der Hülle entspricht;
- Bestimmen der Absorption der Ultraschallwellen durch den Körper (16), allen ausgehend von den in dem genannten zeitlichen Fenster (29) empfangenen Signalen.

12. Verfahren der Messung und/oder der Charakterisierung der Textur bzw. Struktur eines Körpers (16), dadurch **gekennzeichnet**, daß es die Schritte hat, bestehend aus dem:

- Erzeugen einer Aufeinanderfolge von Ultraschallvibrationen bzw. -schwingungen in dem Körper (16), von dem man die Textur bzw. Struktur messen oder charakterisieren will;
- Einfangen der von dem Körper (16) ausgesandten Ultraschallwellen;
- Berechnen einer Aufeinanderfolge von Hüllen (17) der empfangenen Signale (S);
- Berechnen einer mittleren Hülle der empfangenen Hüllen (17);
- Berechnen des neperischen Logarithmus (28) der Hülle des empfangenen Signals;
- Berechnen der linearen Regression (R) des neperischen Logarithmus (28) der Hülle (17) des empfangenen Signals (S) für verschiedene Teile dieser Hülle;
- Ableiten und Quantifizieren des Vorhandenseins von Löchern, Öffnungen und/oder Heterogenitäten in dem genannten Körper, ausgehend von dem Wert des Koeffizienten der Regression.

13. Verfahren gemäß irgendeinem der Ansprüche 7 bis 12, dadurch **gekennzeichnet**, daß das Verfahren ein Verfahren der Messung und/oder der Charakterisierung der Textur bzw. Struktur eines Lebensmittelprodukts, insbesondere eines Käses, ist.

14. Verfahren gemäß irgendeinem der Ansprüche 7 bis 13, dadurch **gekennzeichnet**, daß es einen Schritt der Markierung des Körpers (16), dessen Textur bzw. Struktur gemessen oder charakterisiert worden ist oder eines akustischen Kupplers (15), der in permanenter Art und Weise auf diesem Körper aufgesetzt bzw. angeklebt ist, mit der Sonde (2), insbesondere durch einen Aufdruck bzw. Eindruck bzw. Stempel (49) der Markierungsmittel (48) der Sonde (2), hat.

## Claims

1. A device for measuring and/or characterising the texture of a body (16), characterised in that it comprises a probe (2) for the emission and reception of ultrasonic waves, connected to an emitter (5) for generating an electrical signal converted into ultrasonic waves by the probe (2) and a receiver (6) of the ultrasonic waves emanating from the said body (16) and picked up by the probe (2) and means of processing the signal (7, 8) in order to determine the attenuation in the said body (16) of the ultrasonic waves as a function of the reception time and therefore the depth of penetration into the body (16) and in order to produce therefrom the texture and in particular the hardness of the said body (16).

2. A device according to claim 1, characterised in that the probe (2) furthermore comprises means (13, 14) for measuring the temperature of the body (16) and in that the means of processing the signal (7, 8) take account of the temperature of the body (16) at the time of determination of the texture from the results of the measurement of absorption of the ultrasonic waves.

3. A device according to claim 1 or 2, characterised in that the means of processing the signal (7, 8) calculate on the basis of a succession of ultrasonic signals picked up by the probe (2), a succession of envelopes of the signals received from the said body in order to deduce therefrom a mean envelope on a basis of which the attenuation of ultrasonic waves in the said body is determined.

4. A device according to any one of the preceding claims, characterised in that the device for processing the signal (7, 8) automatically selects a time window (29) in the signal picked up by the probe (2) which has the best signal/noise ratio.

5. A device according to claim 4, characterised in that the means of processing the signal (7, 8) calculate:

- the natural logarithm of the envelope of the signal received;
- the linear regession (R) of the logarithm (28) of the envelope (17) of the signal received for the various parts ofthis envelope;

and determine the time window (29) of the envelope (17) which corresponds to the maximum linear regression coefficient (R) of the logarithm (28) of the envelope and in that the means of processing the signal (7, 8) determine the absorption of ultrasonic waves solely in the said time window (29).

6. A device according to any one of the preceding claims, characterised in that the probe (2) comprises means (48) of marking bodies (16) of which the texture is measured and/or characterised, or an acoustic coupler (15) permanently fixed on this body (16).

7. A method of measuring and/or characterising the texture, particularly the hardness, of the body (16), characterised in that it comprises the stages consisting of:

- generating ultrasonic vibrations in a body (16);
- picking up the ultrasonic waves emitted by the body (16);
- determining the attenuation of ultrasonic waves as a function of the reception time and depth of penetration into the body (16);
- deducing the texture of the body, particularly the hardness of the body (16), from the attenuation of the ultrasonic waves as a function of time in the body (16).

8. A method according to claim 7, characterised in that it comprises a stage involving determination of the temperature of the body (16) of which the texture is being measured or characterised in that determination of the texture, particularly the hardness on a basis of the attenuation of ultrasonic waves in the body (6) takes into account the temperature ofthe said body (16).

9. A method according to claim 7 or 8, characterised in that it comprises stages consisting of:

- generating a succession of ultrasonic vibrations in the body (16) of which it is desired to measure or characterise the texture;
- picking up the ultrasonic signals emitted by the body (16);
- calculating a succession of envelopes (17) of the signals received (S);
- calculating a mean envelope from the envelopes received (17);
- determining the attenuation of ultrasonic waves in the said body (16) on a basis of the said mean envelope.

10. A method according to any one of claims 7 to 9, characterised in that it comprises a stage involving selection of a time window (29) in the signals picked up (S) by the probe (2) and which has the best signal/noise ratio.

11. A method according to claim 10, characterised in that it comprises the stages consisting of

- calculating the natural logarithm (28) of the envelope of the signal received;
- calculating the linear regression (R) of the natural logarithm (28) of the envelope (17) of the signal (S) received for various parts of the said envelope;
- determining the time window (29) of the envelope (17) corresponding to the maximum linear regression coefficient (R) of the natural logarithm (28) and of the envelope;
- determining the absorption of ultrasonic waves by the body (16) solely on a basis with the signals received in the said time window (29).

12. A method of measuring and/or characterising the texture of a body (16) characterised in that it comprises the stages consisting of:

- generating a succession of ultrasonic vibrations in the body (16) of which it is desired to measure or characterise the texture;
- picking up the ultrasonic signals emitted by the body (16);
- calculating a succession of envelopes (17) of the signals received (S);
- calculating a mean envelope from the envelopes received (17);
- calculating the natural logarithm (28) of the envelope of the signal received;

- calculating the linear regression (R) of the natural logarithm (28) of the envelope (17) of the signal (S) received for various parts of this envelope;
- deducing and quantifying the presence of holes, apertures and/or absence of consistency in the said body on a basis of the value of the coefficient of regression.

13. A method according to any one of claims 7 to 12, characterised in that the said method is a method of measuring or characterising the texture of a food product, particularly a cheese.

14. A method according to any one of claims 7 to 13, characterised in that it comprises a stage involving the probe (2) marking the body (16) of which the texture has been measured or characterised or of an acoustic coupler (15) permanently fixed to the said body, particularly by an imprint (49) of the marking means (48) of the probe (2).

FIG.1

## FIG.2

5 ─ Emetteur

8 ─ ─12

─10

UC

Stockage

─11

6 ─ Recepteur

Affichage

9

Conditionneur ─7

2

3 ─15

─16

4

## FIG.3

2

3 ─ ─13

## FIG.4

14 Mesure de Temperature

5 ─ Emetteur

8 ─ ─12

UC

─10 Stockage

─11

6 ─ Recepteur

Affichage

9

Conditionneur ─7

2

15 3 ─13

─16

4

FIG.5

FIG.6

FIG.18

EP 0 578 539 B1

FIG.7

Sonde — Recepteur — A/N — Cadrage (t) — TPR — a+jb — TFR — Enveloppe

2 6 19 21 22 23 24 25 26 20 7

FIG.8

Sonde — Recepteur — Enveloppe

2 6 26 27 7

FIG.9

FIG.10

FIG.11

FIG.12

FIG.13

FIG.14

FIG.15

FIG.16

FIG.17